(19) **Europäisches Patentamt / European Patent Office / Office européen des brevets**

(11) **EP 4 115 746 B1**

(12) **EUROPEAN PATENT SPECIFICATION**

(45) Date of publication and mention of the grant of the patent:
**16.08.2023 Bulletin 2023/33**

(21) Application number: **22184313.9**

(22) Date of filing: **20.11.2020**

(51) International Patent Classification (IPC):
**A24B 13/00** *(2006.01)* **A24B 15/16** *(2020.01)*
**A24B 15/28** *(2006.01)* **A24B 15/30** *(2006.01)*

(52) Cooperative Patent Classification (CPC):
**A24B 13/00; A24B 15/16; A24B 15/285;
A24B 15/30**

(54) **AN ORAL POUCHED NICOTINE PRODUCT INCLUDING A FILLING MATERIAL COMPRISING NICOTINE PARTICLES**

ORALES, IN BEUTELN VERPACKTES NIKOTINPRODUKT MIT EINEM FÜLLMATERIAL MIT NIKOTINPARTIKELN

PRODUIT DE NICOTINE EN SACHET ORAL COMPORTANT UN MATÉRIAU DE REMPLISSAGE COMPRENANT DES PARTICULES DE NICOTINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2019 EP 19210423**

(43) Date of publication of application:
**11.01.2023 Bulletin 2023/02**

(62) Document number(s) of the earlier application(s) in accordance with Art. 76 EPC:
**20807773.5 / 4 061 155**

(73) Proprietor: **Swedish Match North Europe AB
118 85 Stockholm (SE)**

(72) Inventor: **KINDVALL, Mårten
415 24 Göteborg (SE)**

(74) Representative: **Valea AB
Box 1098
405 23 Göteborg (SE)**

(56) References cited:
**US-A1- 2004 043 964    US-A1- 2008 308 115
US-A1- 2010 300 464    US-A1- 2011 083 680
US-A1- 2019 083 393    US-A1- 2019 174 812**

**Description**

TECHNICAL FIELD

The present disclosure relates to an oral pouched nicotine product comprising a filling material and a saliva-permeable pouch of a packaging material enclosing the filling material. The present disclosure also relates to a method for preparing the aforementioned filling material, a method for preparing the aforementioned oral pouched nicotine product as well as to the use of the aforementioned filling material for preparing an oral pouched nicotine product.

BACKGROUND

Traditionally, oral smokeless tobacco products are used in the oral cavity of a consumer to provide nicotine satisfaction from the tobacco in the product. In addition to the tobacco, the oral smokeless tobacco product generally comprises water, salt, pH adjusting agent(s) and additional ingredients such as flavours and humectants. Commonly, these products are called snuff. The snuff may be dry or moist, and may be provided in loose form or in pouched form. Moist snuff is divided into two types, namely American snuff and Scandinavian snuff. American moist snuff is commonly produced through a fermentation process of moisturized ground or cut tobacco. Scandinavian-type moist snuff (snus) is commonly produced using a heat-treatment process (pasteurization) instead of fermentation. The heat treatment is carried out in order to degrade, destroy or denature at least a portion of the organisms within the tobacco preparation.

Oral pouched nicotine products comprising no tobacco or a small amount of tobacco are now becoming increasingly popular among consumers due to inter alia their appealing appearance, freshness and taste. Moreover, this kind of product allows a user to enjoy nicotine without being exposed to tobacco.

Oral pouched nicotine products are typically used by the consumer by placing the pouch between the upper or lower gum and the lip and retaining it there for a limited period of time. The product is configured to fit comfortably and discreetly in the user's mouth. The pouch material holds the filling material in place allowing saliva to pass into the filling material and allowing flavours and nicotine to diffuse from the filling material into the consumer's mouth.

The filling material of such oral smokeless non-tobacco snuff products or oral smokeless low tobacco snuff products may comprise nicotine or lack nicotine.

EP 2 177 213 discloses a nicotine-containing granulate comprising a homogenous mixture of 1-50 % nicotine and 0-99 % excipient, wherein the granulate has a particle size of at least 150 $\mu$m. The excipient may be cellulose. The nicotine-containing granulate is dust-reduced or dust-free due to the presence of the excipient which imparts cohesion to the granulate particles. It is mentioned that the nicotine-containing granulate may be used for the preparation of a pharmaceutical product for oral administration.

EP 1 338 288 discloses cellulose particles for pharmaceutical use, which contain microcrystalline cellulose having an average degree of polymerization of from 60 to 350 in an amount of not less than 10%, and which have a tapped bulk density of from 0.60 to 0.95 g/ml, an aspect ratio of not less than 0.7, a shape factor of from 1.10 to 1.50, and an average particle size of from 10 to 400 mm. Preferably, the particle size is from 40 to 400 $\mu$m, more preferably from 50 to 400 $\mu$m, much more preferably from 50 to 300 $\mu$m, and particularly preferably from 50 to 200 $\mu$m. The particles may be granules containing a medicine inside or on their surface.

US 2019/0083393 discloses use of a nicotine-cellulose composition for the preparation of a snuff composition for achievement of a fast onset of action of nicotine after application of the snuff composition to the oral cavity of a subject. The snuff composition comprises a carrier such as microcrystalline cellulose, which may be selected from AVICEL ® grades.

While powders and granulates are useful in many applications, the small size, irregular shape and/or low fluidity of the powder or granulate particles are frequently associated with special requirements in connection with manufacture to e.g. suppress dust formation and/or achieve satisfactory speed of packaging into e.g. oral pouches. Further, products such as oral pouches comprising powder or granulate may become compressed upon use in the oral cavity thereby changing their shape and/or ease of adjustment to a shape that is complimentary to a location in the oral cavity where it is placed.

There is thus a need for an oral nicotine-containing product involving little or no dust, which allows for release of part or most or all of its nicotine, which provides a pleasant mouth feeling and/or which substantially retains its size and mouth feeling during use in the oral cavity.

SUMMARY

It is an object of the present disclosure to alleviate at least one of the problems discussed above, and/or to provide advantages and aspects not provided by hitherto known technique.

Further, it is an object of the present disclosure to provide an oral pouched nicotine product which provides a pleasant mouth comfort, mouthfeel and/or organoleptic experience of a consumer during oral use.

Another object of the present disclosure is to provide an oral pouched nicotine product having a satisfactory release rate of nicotine in the oral cavity under normal user conditions.

Yet another object of the present disclosure is to reduce or eliminate dust formed when preparing a pouch for oral use comprising the filling material as described herein.

The present disclosure provides an oral pouched nicotine product comprising a filling material and a saliva-permeable pouch of a packaging material enclosing the filling material,

said filling material comprising:

(a) tobacco-free nicotine-containing particles formed from a carrier material,

said carrier material comprising microcrystalline cellulose,
said tobacco-free nicotine-containing particles comprising a nicotine source,
said tobacco-free nicotine-containing particles having
a sphericity from about 0.8 to about 1.0,
a diameter from about 0.7 mm to about 1.0 mm,
a d50 value from 0.9 mm to 1.0 mm, and
a moisture content from 8 wt% to 15 wt% based one the total weight of the tobacco-free nicotine-containing particles, and
(b) optionally a tobacco material present in an amount within the range of from about 0.05 wt% to about 10 wt%, based on the total weight of said filling material
with the proviso that said oral pouched nicotine product is free from or substantially free from a constituent comprising a coating comprising a nicotine source and a binder.

The present disclosure also provides a method for preparing an oral pouched nicotine product as described herein, said method comprising the steps of:

a) providing a filling material as described herein, and
b) enclosing said filling material in a saliva-permeable pouch.

The present disclosure also provides a use of a filling material as described herein for preparing an oral pouched nicotine product.

The present disclosure also provides a method for preparing the filling material as described herein, the method comprising the steps of:

a) providing nicotine-free particles formed from a carrier material comprising or consisting of microcrystalline cellulose,
b) providing a mixture comprising a nicotine source, a solvent such as water, and optionally an additive,
c) subjecting the particles of step a) to the mixture of step b) whereby part or all of the mixture is included in the nicotine-free particles from step a) thereby providing tobacco-free nicotine-containing particles,
d) optionally adding a flavouring agent to the particles resulting from step c),
e) optionally adding nicotine-free additional particles to the particles obtained in steps c) or d), and
f) optionally changing the moisture content of the particles in any one of steps a) to e).

DEFINITIONS

The term "tobacco material" is used herein for fibrous material of tobacco leaves, parts of leaves, such as lamina and stem, or tobacco extract. The leaves and parts of leaves may be finely divided (disintegrated), such as ground, cut, shredded or threshed, and the parts of leaves may be blended in defined proportions in the tobacco material.

By "tobacco" as used herein is meant any part, e.g. leaves, stems, and stalks, of any member of the genus *Nicotiana.* The tobacco may be whole, shredded, threshed, cut, ground, cured, aged, fermented, or treated otherwise, e.g., granulated or encapsulated.

The term "nicotine" refers to nicotine in any form, such as liquid or solid form.

As used herein the terms "pouched nicotine product for oral use" or "oral pouched nicotine product" and the like refer to a portion of nicotine-containing filling material packed in a saliva-permeable pouch material intended for oral use. Two examples of oral pouched nicotine products are oral pouched nicotine non-tobacco products and oral pouched low tobacco nicotine products.

As used herein the terms "oral pouched nicotine non-tobacco product", "oral pouched tobacco free nicotine product" or "oral pouched nicotine product free from tobacco" and the like refer to a portion of nicotine-containing filling material packed in a saliva-permeable pouch material intended for oral use wherein no tobacco is included in said product.

As used herein the term "oral pouched low tobacco nicotine product" and the like refers to a portion of nicotine-containing filling material packed in a saliva-permeable pouch material intended for oral use wherein an amount of tobacco material within the range of from about 0.1% to about 10% by weight or from about 0.1% to about 5% by weight, based on the total weight of the filling material, is included in said product.

As used herein, the term wt% stands for weight %. In this document, wt%, weight % and % by weight are interchangeable.

As used herein, the term mm stands for millimetre(s).

As used herein, the term "moisture content" refers to the total amount of oven volatile ingredients, such as water and other oven volatiles (e.g. propylene glycol) in the preparation, composition or product referred to. The moisture content is given herein as percent by weight (wt%) of the total weight of the preparation, composition or product referred to.

The moisture content as referred to herein may be determined by using a method based on literature references Federal Register/ vol.74, n o. 4/712-719/Wednesday, January 7, 2009/Notices "Total moisture determination" and AOAC (Association of Official Analytical Chemics), Official Methods of Analysis 966.02: "Moisture in Tobacco" (1990), Fifth Edition, K. Helrich (ed). In this method, the moisture content is determined gravimetrically by taking $2.5 \pm 0.25$ g sample and weighing the sample at ambient conditions, herein defined as being at a temperature of 22°C and a relative humidity of 60%, before evaporation of moisture and after completion of dehydration. Mettler Toledo's Moisture Analyzer HB43, a balance with halogen heating technology, is used (instead of an oven and a balance as in the mentioned literature references) in the experiments described herein. The sample is heated to 105°C (instead of $99.5 \pm 0.5$°C as in the mentioned literature references). The measurement is stopped when the weight change is less than 1 mg during a 90 seconds time frame. The moisture content as weight percent of the sample is then calculated automatically by the Moisture Analyzer HB43.

"Flavour" or "flavouring agent" is used herein for a substance used to influence the aroma and/or taste of the nicotine product, including, but not limited to, essential oils, single flavour compounds, compounded flavourings, and extracts.

The term "d50" or "d50 value" used herein refers to the value of the particle diameter in a cumulative distribution_of a group of particles with respect to volume. For example, if d50=0.8 $\mu$m, then 50% of the particles in the sample are larger than, i.e. have a diameter that is larger than, 0.8 $\mu$m, and 50% smaller than, i.e. have a diameter that is less than, 0.8 $\mu$m. In a further example, if d10=0.8 mm, then 10% of the particles have a diameter that is less than 0.8 $\mu$m and 90% of the particles have a diameter that is less than 0.8 $\mu$m.

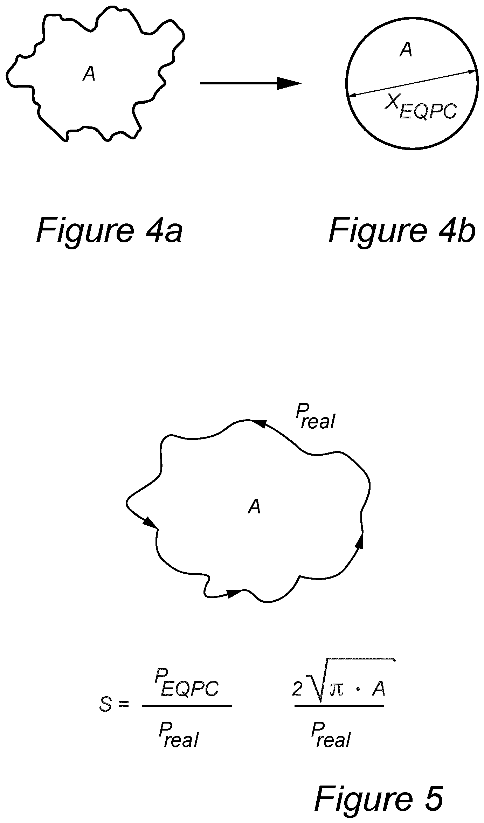

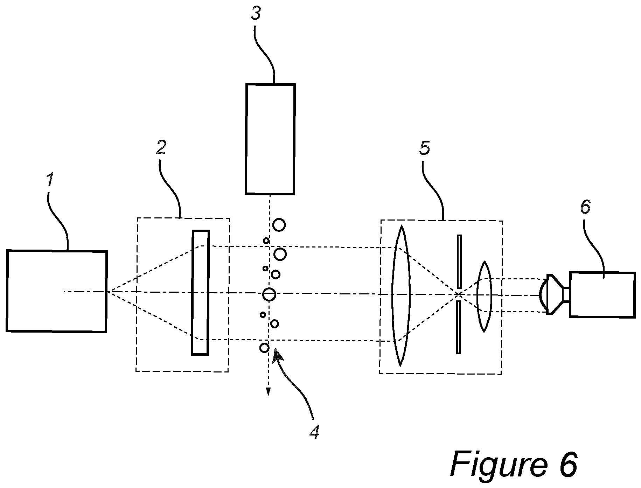

The sphericity, which may be denominated "$S$", is a measurement of how close a particle is to be a mathematically perfect sphere. The sphericity $S$ is the ratio of the perimeter of the equivalent circle, $P_{EQPC}$, to the real perimeter, $P_{real}$. The perimeter of the equivalent circle, $P_{EQPC}$, is the perimeter of a circle that has the same area as the projection area of the particle using e.g. image analysis. In this context, the projection area is the area resulting from a two-dimensional projection of the three-dimensional particle. The sphericity value S may be from 0.5 to 1. A particle having a sphericity value S equal to 1 has the shape of a sphere. The sphericity may be calculated based on a measurement using image analysis on a sample of particles, wherein the projected area $A$ and the projected real perimeter $P_{real}$ are and recorded. The particles may be projected in the same plane. For instance, the particles may be dispersed in a dispersing unit and then measured as shown in Figure 6. The image analysis may be performed using an instrument from Sympatec as described herein. The sphericity may be calculated by equation (1) which is also shown in Figure 5.

Equation (1):

$$S = \frac{P_{EQPC}}{P_{real}} = \frac{2\sqrt{\pi \cdot A}}{P_{real}}$$

The diameter of the particles described herein, such as the nicotine containing particles, may be the diameter of a circle of equal projection area. This is the diameter of a circle that has the same area as the projection area of the particle. In this context, the projection area is the area resulting from two-dimensional projection of the three-dimensional particle. As used herein, $x_{EQPC}$ refers to the diameter of a circle of equal projection area. Figure 4a shows a two-dimensional projection of a three dimensional particle having a projection area $A$. Figure 4b shows the two-dimensional projection of Figure 4a converted into a circle of equal, i.e. the same, projection area $A$ as the projection of Figure 4a. The diameter of the circle of equal projection area, $x_{EQPC}$, is indicated in the circle of Figure 4b. Image analysis may be used for determining the diameter of the particles described herein. For instance, the image analysis may be performed using an instrument from Sympatec as described herein. Additionally or alternatively, the diameter of the particles described herein may be characterized by the mesh size of a mesh through which the particles may pass. For instance, the particles described herein may pass through a mesh having a mesh size from about 140 to about 10. This corresponds to a mesh size range from about 0.10 millimeters to about 2.0 millimeters.

As used herein, the expression "included in" intends "absorbed in and/or adsorbed onto". For example, "included in the carrier material" intends "absorbed in and/or absorbed onto the carrier material".

BRIEF DESCRIPTION OF THE DRAWINGS

Figure 1 shows the particle size distribution of Cellets 700 particles.
Figure 2 shows the flow rates for a powder and for Cellets 700 particles, respectively.
Figure 3 shows the compression of Cellets 700 particles and MCC Avicel PH200, respectively.
Figure 4a shows a two-dimensional projection of a three dimensional particle having a projection area $A$.
Figure 4b shows a circle of equal projection area $A$ as the particle of Figure 4a.
Figure 5 shows an equation for calculating sphericity for a particle having a real perimeter $P_{real}$ and a projection area $A$.
Figure 6 shows an image analysis instrument for measurement of particles.

DESCRIPTION

The present disclosure provides an oral pouched nicotine product comprising a filling material and a saliva-permeable pouch of a packaging material enclosing the filling material,

said filling material comprising:

(a) tobacco-free nicotine-containing particles formed from a carrier material,

said carrier material comprising microcrystalline cellulose,
said tobacco-free nicotine-containing particles comprising a nicotine source,
said tobacco-free nicotine-containing particles having
a sphericity from about 0.8 to about 1.0,
a diameter from about 0.7 mm to about 1.0 mm,
a d50 value from 0.9 mm to 1.0 mm, and
a moisture content from 8 wt% to 15 wt% based one the total weight of the tobacco-free nicotine-containing particles, and
(b) optionally a tobacco material present in an amount within the range of from about 0.05 wt% to about 10 wt%, based on the total weight of said filling material
with the proviso that said oral pouched nicotine product is free from, i.e. does not contain, or is substantially free from, i.e. does not substantially contain, a constituent comprising a coating comprising a nicotine source and a binder.

Thus, it is appreciated that the oral pouched nicotine product described herein may be free from any constituent comprising a coating comprising a nicotine source and a binder, i.e. it does not contain a constituent comprising a coating comprising a nicotine source and a binder. Accordingly, the tobacco-free nicotine-containing particles of the filling material may not comprise a coating comprising a nicotine source and a binder.

Alternatively, the oral pouched nicotine product described herein may comprise a constituent comprising a coating comprising a small amount, such as from about 0.1 to about 1 wt %, nicotine source and a binder, i.e. it does not substantially contain a constituent comprising a coating comprising a nicotine source and a binder. For example, the tobacco-free nicotine-containing particles may include most or substantially all of the nicotine such as the nicotine source within the carrier material and only a small amount of the nicotine present in in the coating comprising a nicotine source and a binder.

The carrier material described herein may be a matrix material, a dense material or a combination thereof. The matrix material may comprise internal cavities such as pores. Thus, the matrix material may be porous. The material such as the dense material may lack or substantially lack pores. Thus, the dense material may be non-porous. The carrier material may comprise or consist of microcrystalline cellulose. For instance, the carrier material may comprise or consist of microcrystalline cellulose and optionally a saliva-soluble and/or saliva insoluble material as described herein.

The tobacco-free nicotine-containing particles described herein may be non-dried, i.e. they have not been subjected to drying such as drying involving or lacking application of heat. It is believed that lack of drying beneficially affects the components included in said particles, such as the nicotine source and/or the flavouring agent, which may degrade and/or be altered by drying. It will be appreciated that mere evaporation of a small amount of solvent or volatile components, such as from about 1 wt% to about 25 wt% of solvent and/or volatile components, is not regarded as drying.

The nicotine source may be included in the carrier material of the tobacco-free nicotine-containing particles. In other words, the nicotine source may be absorbed in the carrier material and/or adsorbed on an outer surface of the carrier material. For example, the nicotine source may be present in the internal cavities such as pores of the carrier material. Additionally or alternatively, the nicotine source may be distributed in channels within the carrier material. Further, at least part of the nicotine source may be located on an outer surface of the tobacco-free nicotine-containing particles. For example, most of the nicotine source, such as about 95 wt% to about 99.5 wt% of the nicotine source, may be absorbed in the carrier material and a small part of the nicotine source, such as about 0.5 wt% to about 5 wt%, may be located on an outer surface of the carrier material. As described herein, the tobacco-free nicotine-containing particles change colour upon storage which indicates that at least a part of the nicotine source is located on an outer surface of the tobacco-free nicotine-containing particles. Further, this indicates that the nicotine source absorbed in the carrier material is close to the outer surface of the tobacco-free nicotine-containing particles rather than being distributed evenly throughout the carrier material. Thus, it appears that there is a concentration gradient of the nicotine source in the carrier material wherein most of the absorbed nicotine source is close to the outer surface of the carrier material of the tobacco-free nicotine-containing particles.

Thus, there is provided an oral pouched nicotine product according to any one of the preceding claims, wherein the tobacco-free nicotine-containing particles change colour or colour shade upon storage

> such as storage taking place
> at a relative humidity from about 60% to about 75%,
> at a temperature from about 22°C to about 30 °C, and/or
> for a time of about 15 weeks.

The change in colour or colour change described herein may be determined by visual inspection using the naked eye. Additionally or alternatively, an instrument such as a spectrophotometer for measuring change in colour or colour shade may be used. For example, a spectrophotometer from ColorFlex EZ, Hunter Lab, may be used.

The shape of the filling material particles may be spherical or substantially spherical. Spherical particles are understood to have a sphericity of 1.0 or of about 1.0. As used herein, the expressions "filling material particles" or "particles of the filling material" intend the tobacco-free nicotine-containing particles and/or the nicotine-free additional particles described herein. Substantially spherical particles are understood to have a sphericity below 1.0 such as from about 0.80 to about 0.95. The sphericity may be determined as described herein. The filling material particles may have the same or substantially the same sphericity. For example, the tobacco-free nicotine-containing particles and the nicotine-free additional particles may have the same or substantially the same sphericity. Alternatively, the sphericity of the tobacco-free nicotine-containing particles and the nicotine-free additional particles may be different. As used herein, the expression "the same or substantially the same sphericity" intends particles varying in sphericity by $\pm 30\%$ or less, such as $\pm 20\%$ or less, such as $\pm 10\%$ or less.

The particles of the filling material as described herein may all have the same or substantially the same size. As used herein, the expression "the same size or substantially the same size" intends particles varying in diameter by $\pm 30\%$ or less, such as $\pm 20\%$ or less, such as $\pm 10\%$ or less. The particle size described herein may be measured by image analysis. For instance, image analysis may be performed using an instrument from Sympatec as described herein. For non-spherical particles the diameter may be the diameter of a circle of equal projection area; i.e. the diameter of a circle that has the same area as the two-dimensional projection area of the particle.

The nicotine-free additional particles of the filling material may have a diameter from about 0.15 mm to about 2.0 mm, such as from about 0.3 mm to about 2.0 mm, such as from about 0.4 mm to about 2.0 mm, such as from about 0.5 mm to about 2.0 mm such as from about 0.6 mm to about 2.0 mm, such as from about 0.7 mm to about 2.0 mm, such as from about 0.8 mm to about 2.0 mm, such as from about 0.9 mm to about 2.0 mm, such as from about 0.7 mm to about 1.5 mm, such as from about 0.9 mm to about 1.2 mm, such as from about 0.7 to about 1.1 mm. For instance, the nicotine-free additional particles of the filling material may have a diameter from about 0.7 mm to about 1.1 mm. The particle diameter may be determined using image analysis using e.g. a Sympatec instrument as described herein. The tobacco-free nicotine-containing particles and the nicotine-free additional particles may have the same diameter or different diameters.

As explained herein, the particle analysis may be performed using image analysis such as with an instrument from Sympatec GmbH, Germany. Such an instrument may operate as shown in Figure 6, wherein 1 is a pulsed light source, 2 is a beam expansion unit adaptable to measuring range, 3 is a dispersing unit, 4 is particle flow, 5 is an objective and 6 is a camera. A well dispersed particle flow is led through the image plane. The particles are separated from each other by a transportation fluid and overlapping particles are avoided. A high number of particle numbers per image frame may be captured.

In an example, the nicotine-free additional particles of the filling material particles may have a sphericity from about 0.8 to about 1.0 and/or a particle diameter from about 0.2 mm to about 1.8 mm, such as from about 0.2 mm to about 1.5 mm, such as from about 0.2 mm to about 1.0 mm, such as from about 0.2 mm to about 0.5 mm. In a further example, the filling material particles may have a sphericity from about 0.80 to about 0.85, from about 0.85 to about 0.90, from about 0.90 to about 0.95, or from about 0.95 to about 1.0. In still a further example, the particles of the filling material may have a sphericity from about 0.9 to about 1.0 and/or a diameter from about 0.7 to about 1.0. The tobacco-free nicotine-containing particles and the nicotine-free additional particles may have the same sphericity or different sphericities.

Further, the nicotine-free additional particles of the filling material may have a d50 value within the range of from about 0.15 mm to about 1.5 mm, such as from about 0.7 mm to about 1 mm, such as from about 0.8 to about 1 mm, such as about 0.7 mm, such as about 0.8 mm, such as about 0.9 mm, such as about 1.0 mm, such as from 0.9 mm to 1.0 mm. In an example, the particles of the filling material may have a d50 value from about 0.9 to about 1.0. The tobacco-free nicotine-containing particles and the nicotine-free additional particles may have the same d50 value or different d50 values.

In an example, the nicotine-free particles may have a diameter within the range of from about 0.8 mm to about 1.15 mm and have a d50 of about 0.9. In a further example, the d50 value may be about 0.2, 0.3, 0.5, 0.6, 1.2, 1.5 or 2.0 mm. This value may vary by ±30% or less, such as ±20% or less, ±10%or less, or ±5% or less. In a further example, the nicotine-free particles may have a diameter from about 0.7 mm to about 1.0 mm, a d50 value from about 0.9 to about 1.0, and a moisture content from about 8 wt% to about 15 wt% based one the total weight of the tobacco-free nicotine-containing particles or the nicotine-containing particles.

The outer surface of the particles and/or particle core(s) may be smooth or at least substantially smooth. For instance, the particles may be considered to exhibit a smooth outer surface if they exhibit a flow rate equal to or above about 2 grams/second, such as from about 2 grams/second to about 10 grams/second, and/or an angle of repose equal to or less than about 41°, such as from about 35° to about 41°. As used herein, the angle of repose is the steepest angle of descent or dip relative to the horizontal plane to which a material can be piled without slumping. At this angle, the material is on the verge of sliding. The angle of repose may be measured as known in the art. For example, it may be measured using the tilting box method, the fixed funnel method and/or the revolving cylinder method.

The particles of the filling material described herein have been found to have a better fluidity as compared to the fluidity of a powder. As a result, an oral pouched nicotine product enclosing said particles may easily adjust its shape to fit onto or into an object that is contacted with the product such as a place within the oral cavity. Further, the particles of the filling material are associated with good handling properties such as good packaging properties enabling e.g. convenient filling into a pouch for oral use. The good handling properties also involve *inter alia* formation of less dust as compared to powders or granulates. Further, the oral pouched nicotine product may be perceived as less dry as compared to an oral pouched nicotine product comprising a powder.

In an example, the particles of the filling material have a flow rate that is equal to or above about 2 grams/second, such as from about 2 grams/second to about 10 grams/second, and/or an angle of repose that is equal to or less than about 41° such as about 35° or less, such as from about 35° to about 41°. The flow rate may be measured as described herein or as known in the art. The angle of repose may be measured as described herein.

The pouch of the oral pouched nicotine product may be made of any suitable saliva-permeable, and preferably non-dissolvable, packaging material, such as a non-woven material. The packaging material - herein also called pouch material - may be a nonwoven material comprising staple fibres of regenerated cellulose, such as viscose rayon staple fibres, and a binder, such as a polyacrylate. The packaging material may also comprise additional ingredients, such as flavouring agents and/or colorants. Further, the packaging material may be a pharmaceutically acceptable material.

In an example, the packaging material of the pouch described herein comprises or consists of a nonwoven material, wherein said packaging material comprises a nonwoven material comprising fibres, said fibers having a linear density that are less than about 1.7 decitex, and said nonwoven material having a basis weight equal to or above about 30 g/m2. This allows the packaging material to exhibit satisfactory strength, flexibility and/or permeability for saliva and the filling material components.

The carrier material of the tobacco-free nicotine-containing particles may consist of microcrystalline cellulose. Alternatively, the carrier material of the tobacco-free nicotine-containing particles described herein may comprise microcrystalline cellulose, and optionally a saliva-soluble material and/or a saliva non-soluble material. The saliva soluble material and/or saliva insoluble material may be selected from the group consisting of cellulose, starch, polyalcohol and sugar. For instance, the carrier material of the tobacco-free nicotine-containing particles may comprise microcrystalline cellulose and optionally isomalt, sugar and/or maltitol. In still a further example, there is provided a mixture of (i) tobacco-free nicotine-containing particles wherein the carrier material consists of microcrystalline cellulose and (ii) tobacco-free nicotine-containing particles wherein the carrier material comprises microcrystalline cellulose, a saliva-soluble material and/or a saliva non-soluble material.

The microcrystalline cellulose of the particles described herein such as the tobacco-free nicotine-containing particles may constitute from about 50 wt% to about 100 wt% of the carrier material based on the total weight of the carrier material. For example, the microcrystalline cellulose may constitute from about 70 wt% to about 100 wt%, such as from about 80 wt% to about 100 wt%, such as from about 90 wt% to about 100 wt%, of the carrier material based on the total weight of the carrier material.

The tobacco-free nicotine-containing particles and/or the nicotine-free additional particles described herein may comprise one or more nicotine-free coating(s), i.e. coating(s) lacking nicotine such as a nicotine source as described herein. The one or more coatings may have a uniform or non-uniform thickness. In an example, the one or more coatings comprise(s) a uniform or substantially uniform thickness in the range from about 10 μm to about 100 μm, such as from about 25 μm to about 50 μm. The application of the one or more nicotine free coating(s) allows for providing a substantially smooth particle outer surface, a desired particle shape such as a spherical or substantially spherical shape and/or a desired fluidity. When placed in the oral cavity of a consumer, the one or more coating(s) may be released and/or dissolved without crushing the nicotine containing particles of the filling material so that mastication and/or chewing is not required.

Alternatively, the tobacco-free nicotine-containing particles and/or the nicotine-free additional particles may be free from a coating, i.e. may lack a coating / uncoated.

The one or more nicotine-free coatings described herein may be applied onto at least part of an outer surface, such as the entire outer surface, of the nicotine containing particles by e.g. using a coating fluid bed spraying.

The thickness of the one or more coatings may be small as compared to the nicotine containing particle diameter. For instance, said thickness may be 20% or less of the particle diameter. As a result, the size and/or shape of the nicotine containing particles may remain substantially unchanged when they are contacted with saliva such as human saliva. Therefore, the mouth feeling experienced by a consumer using the product in the oral cavity may remain substantially unchanged over time.

The one or more nicotine-free coating(s) may comprise a binder and optionally an additive as described herein. The binder may be saliva soluble thereby allowing it to be released and/or dissolved without requiring chewing or mastication. The binder may be selected from the group consisting of hydroxypropyl methylcellulose (HPMC), hydroxypropyl cellulose (HPC), methyl cellulose (MC), polyvinylpyrrolidone (PVP) and any mixture thereof. In addition to functioning as a binder, the binder may allow for protecting the nicotine containing particles.

The nicotine source described herein may comprise nicotine base and/or be selected from the group consisting of nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, nicotine benzoate, nicotine polacrilex and any combination thereof. For example, the nicotine source may comprise or consist of nicotine base. In a further example, the nicotine source may comprise one or more of the following: nicotine monotartrate, nicotine bitartrate, nicotine bitartrate dihydrate. In still a further example, the nicotine source may be nicotine bitartrate and/or nicotine bitartrate wtmonohydrate. It will be appreciated that the nicotine source described herein may not comprise tobacco.

The amount of nicotine per oral pouched nicotine product may be within the range from about 0.1 mg to about 20 mg of nicotine calculated as nicotine base, such as about 0.5 mg, about 1.0 mg, about 1.5 mg, about 2.0 mg, about 2.5 mg, about 3.0 mg, about 3.5 mg, about 4.0 mg, about 4.5 mg, about 5.0 mg, about 6.0 mg, about 7.0 mg, about 8.0 mg, about 9.0 mg, about 10 mg, about 12 mg, about 14 mg, about 16 mg, about 18 mg, or about 20 mg of nicotine. For instance, the amount of nicotine may be from about 0.8 wt% to about 2 wt% based on the total weight of the filling material.

The filling material as disclosed herein may further comprise nicotine-free additional particles. These particles may be the same or substantially the same as the tobacco-free nicotine containing particles of the filling material except for the lack of a nicotine source. Alternatively, these particles may be different from the tobacco-free nicotine containing particles of the filling material. The nicotine free additional particles may comprise an additive as described herein, lack an additive as described herein or be a mixture thereof. The size, density and/or shape of these nicotine free additional particles may be the same or substantially the same as for tobacco-free nicotine-containing particles. Advantageously, the density of the nicotine free additional particles may be the same or substantially the same as that of the particles of the filling material since this minimizes the risk for segregation when the oral pouched nicotine product is stored. The nicotine-free additional particles may lack a coating or comprise one or more nicotine-free coatings.

The filling material described herein may further comprise an additive selected from the group consisting of a pH adjusting agent, a flavouring agent, a sweetener, a humectant and any mixture thereof. The additive may be included in the tobacco-free nicotine-containing particle and or the nicotine-free additional particles. In particular, the additive may be a flavouring agent, such as a synthetic flavour, a plant based flavour, a flavour oil, a hydrophobic flavour oil such as an essential oil, such as a nature-identical flavour. As used herein, a nature-identical flavour intends a synthetic flavour which is chemically identical to natural flavourings but are prepared or extracted using chemical methods. The flavouring agent may be a mixture of different flavours. The humectant may be glycerol and/or propylene glycol. The sweetener may be an artificial sweetener.

The additive as disclosed herein may be

- mixed with the filling material,
- included in the tobacco-free nicotine-containing particles,
- included in the one or more nicotine free coatings, and/or
- included in the nicotine-free additional particles.

For instance, the additive such as the flavouring agent may be included in the carrier material of the particles described herein such as the tobacco-free nicotine-containing particles and/or the nicotine-free additional particles. For example, the additive may be present in the internal cavities of the carrier material if such internal cavities are present. Additionally or alternatively, the additive may be distributed within the substance constituting the carrier material. Further, the additive may be located on an outer surface of the particles described herein.

The filling material of the oral pouched nicotine product may comprise a pH adjusting agent. For instance, the pH adjusting agent may be included in the nicotine containing particles of the filling material. Additionally or alternatively, the pH adjusting agent may be included into the nicotine free additional particles described herein. Thus, there is provided an oral pouched nicotine product as described herein, comprising nicotine-free additional particles comprising:

(i) a carrier material comprising or consisting of microcrystalline cellulose,
(ii) a pH adjusting agent;

wherein said pH adjusting agent is included in said microcrystalline cellulose,
said particles having a sphericity from about 0.8 to about 1.0 and a diameter from about 0.1 mm to about 2.0 mm. In an example, there is provided nicotine free-additional particles that are formed from the carrier material, said carrier material comprising or consisting of microcrystalline cellulose, said nicotine-free additional particles comprising a pH adjusting agent, said nicotine free additional particles having a sphericity from about 0.8 to about 1.0 and a diameter from about 0.1 mm to about 2.0 mm.

By keeping a part or all of the pH adjusting agent separate from the nicotine source the storage stability of the nicotine source may be improved since a high pH may have a negative effect on the stability of the nicotine source. Thus, the pH adjusting agent may be present in the nicotine-free particles and absent in the tobacco-free nicotine-containing particles.

The pH adjusting agent of the oral pouched nicotine product as described herein allows the pH to be within the range of from about 7 to about 10. This pH may be achieved after manufacture of the product, such as immediately after manufacture of the product. Further, this pH is provided upon storage of the product such as upon storage at room temperature, or in a refrigerator at approximately from about 5 °C to about 10 °C.

Achieving a pH within the range of about 7 to about 10, such as from about 7 to about 9.5, such as from about 7 to about 9.2, such as from about 7 to about 9, such as from about 8 to about 9, is advantageous since it allows for good nicotine extraction and taste while not impacting oral mucous membranes negatively.

The amount of pH adjusting agent may be selected such that the filling material when dispersed in purified water provides a pH above 7.0, such as a pH within the range of from about 7.0 to about 10.0 or a pH within the range of from about 8.0 to about 9.0, such as a pH within the range of from about 8.3 to about 8.7.

The pH of the filling material can be measured by adding 100 ml of distilled water to 5.0 gram of filling material, for instance in a 100 ml Erlenmeyer flask, stirring the resulting mixture at room temperature with a magnetic stirrer at 100 rpm for about 5 minutes, and then measuring the pH of an extract obtained therefrom with a calibrated (according to the manufacturer's instructions) pH meter. For correctness of readings, the sample solutions shall be analyzed within one hour. In this document, the term "rpm" stands for revolutions per minute. Further, in this document the expression "room temperature" stands for a temperature from about 20°C to about 25°C, such as about 22°C.

Examples of suitable pH adjusting agents include sodium hydroxide, potassium hydroxide, potassium carbonate, sodium carbonate, sodium bicarbonate, potassium bicarbonate, magnesium carbonate and any combination thereof. For instance, the pH adjusting agent may be potassium hydroxide. In a further example, the pH adjusting agent may be sodium carbonate, potassium carbonate, sodium bicarbonate and/or potassium bicarbonate. In still a further example, the pH adjusting agent may comprise or consist of potassium carbonate. The pH adjuster may be included in the tobacco-free nicotine-containing particles and/or the nicotine-free particles described herein. As described herein, it may be advantageous to keep the nicotine source and the pH adjuster apart. For example, the pH adjusting agent may be present in the nicotine-free particles and absent in the tobacco-free nicotine-containing particles.

The filling material may comprise one or more flavouring agents. For instance, the flavouring agent may be a liquid, an oil, a solid such as a particulate material or a mixture thereof. In an example, the flavouring agent is a synthetic flavour, a plant based flavour, a flavour oil, a hydrophobic flavour oil such as an essential oil. The flavouring agent may be a mixture of different flavours.

The flavouring agent may be stable at pH > 7. Further, the flavouring agent of the filling material in the oral pouched nicotine product as described herein may be a hydrophobic flavouring agent such as a hydrophobic flavour oil.

Examples of flavours of the flavouring agent(s) include bergamot, eucalyptus, orange, mandarin, citrus, lemon, peppermint, spearmint, mint, menthol, liquorice, wintergreen, whiskey, rum, cherry, various berries, tobacco, coffee, vanilla, lime, apple, peach, carvone, limonene and any combination of two or more thereof. In an example, the flavour comprises tobacco flavour. In a further example, the flavour is free from tobacco flavour.

The filling material of the oral pouched nicotine product as described herein may comprise within the range of from about 0.5% to about 3.0% by weight of the flavouring agent, based on the total weight of the filling material.

The flavouring agent may be provided in admixture with the filling material particles such as to form a mechanical mixture or mass, incorporated into the tobacco-free nicotine-containing particles and/or the nicotine-free additional particles, incorporated into one or more nicotine free coatings as described herein and/or added to a container comprising the oral pouched nicotine product described herein.

Addition of the flavouring agent to the oral pouched nicotine product described herein may be achieved by adding said flavouring agent to an inside surface of a container for the oral pouched nicotine product such as a can, and allowing said flavouring agent to diffuse or migrate from the inside surface to the oral pouched nicotine product. Thus, the present disclosure also provides a method for adding a flavouring agent as described herein to an oral pouched nicotine product as described herein, said method comprising the steps of:

- applying the flavouring agent onto at least one inside surface of a container for the oral pouched nicotine product,
- placing the oral pouched nicotine product in the container, and
- closing the container.

It will be appreciated that the filling material may be free from small particles such as particles having a largest dimension equal to or below about 0.7 mm, such as a diameter equal to or below about 0.7 mm. This is advantageous since it reduces problems associated with the presence of small particles such as dust and/or poor fluidity.

The container may be a packaging and/or consumer container suitable for oral pouched nicotine products. Thus, the container described herein may be adapted for being conveniently carried in a consumer pocket or handbag, and/or may also be used for packaging any known type of snuff product such as oral pouched nicotine products as described herein. The container may be made of plastics and/or metal. Further, the container may have any desired shape or geometrical form. For example, the container may have the form of a cylinder. The container may comprise a top and a base defining an interior space. The base may comprise a base surface and surrounding walls extending from said base surface. The top may be in the form of a lid that is detachable from the base of the container, or in the form of a lid that is hinged or otherwise attached to the base of the container. For example, the lid may be attached by to the base by snap-fit. The container may be tamper resistant. In an example, the container may be as described in WO 2017/125405 which is incorporated herein in its entirety. In a further example, the container may be as shown in the Design Registration in Norway No. 085548.

From a manufacturing point of view, it may be desirable to apply the flavouring agent to the bottom inner surface of the base and/or the top inner surface of the lid. The container is thereafter closed.

Flavourization may take place during storage for a certain time such as a week. The storage may take place at room temperature, i.e. from about 20 °C to about 25 °C, or in a refrigerator from about 5 °C to about 10 °C. During the storage time, the flavouring agent diffuses or migrates from the at least one inside surface of the container to the oral pouched nicotine product.

Accordingly, the method for adding a flavouring agent may further comprise a step of: d) storing the container for a week.

The filling material may also comprise a sweetener such as natural or synthetic sweeteners. Examples of sweeteners include sucrose, glucose, dextrose, maltose, fructose, saccharin, aspartame, acesulfame, sucralose, cyclamate and any mixture thereof.

The sweetener may be included in the nicotine containing particles and/or the nicotine free additional particles as described herein. Additionally or alternatively, the sweetener may be provided in admixture with the filling material such as to form a mechanical mixture or mass.

The oral pouched nicotine product may comprise no tobacco, i.e. it may be free from tobacco. Thus, there is provided an oral pouched nicotine product as described herein that is free from tobacco.

Alternatively, the filling material may comprise a tobacco material within the range of from about 0.05% to about 10% by weight, such as from about 0.05% to about 5% by weight, such a from 0.2 t% to 1 wt%, such as from about 0.05% to about 0.5% by weight, such as from 0.05% to about 0.3% by weight, based on the total weight of the filling material. For instance, the filling material may comprise a tobacco material within the range of from about 0,2 wt% to about 1 wt% based on the total weight of the filling material. The tobacco material may be present as loose and/or divided tobacco material. Thus, an oral pouched nicotine product comprising the aforementioned filling material including tobacco may be a low tobacco oral pouched nicotine product. The tobacco material may be mixed with the filling material particles to provide a mixture such as a homogenous mixture. The tobacco material may be a purified tobacco material, such as a bleached tobacco material. Further, the tobacco material may be provided as tobacco fibers, ground tobacco, tobacco extract and/or as snuff such as snus.

The filling material as disclosed herein may further comprise water in an amount from about 1 wt% to about 50 wt%, such as from about 20 wt% to about 45 wt%, such as from about 1 wt% to about 20 wt%, such as about from 1 wt% to about 10 wt%, such as about 20 wt%, such as from about 1 wt% to about 3 wt% based on the total weight of the filling material. In an example, the filling material comprises from about 1 wt% to about 5 wt% of water based on the total weight of the filling material.

The moisture content of the filling material described herein may be within the range of from about 0.5 wt% to about 25 wt%, such as from about 0.5 wt% to about 25 wt%, based on the total weight of the filling material. For example, the filling material may have a moisture content within the range of from about 0.5 wt% to about 25 wt%, such as from about 5 wt% to about 25 wt%, such as from about 8 wt% to about 25 wt%, such as from about 5 wt% to about 15 wt%, such as from 8 about wt% to about 15 wt%, based on the total weight of the filling material. In a further example, the moisture content of the filling material is from about 8 wt% to about 15 wt% based on the total weight of the filling material. In a further example, the tobacco-free nicotine-containing particles and/or the nicotine-free additional particles have a moisture content from about 0.5 wt% to about 25 wt%, such as from about 5 wt% to about 25 wt%, such as from about 8 wt% to about 25 wt%, such as from about 5 wt% to about 15 wt%, such as from about 8 wt% to about 15 wt%, such as from based on the total weight of the filling material.

The the tobacco-free nicotine-containing particles and/or the nicotine-free additional particles may be provided in an amount within the range of from about 50 wt% to about 100 wt%, such as from about 50 wt% to about 90 wt%, based on the total weight of the filling material.

There is also provided an oral pouched nicotine product as described herein, wherein the nicotine free additional particles have a sphericity from about 0.8 to about 1.0, a diameter from about 0.7 mm to about 1.1 mm, a d50 value from about 0.9 mm to about 1.0 mm.

There is also provided nicotine-containing particles as disclosed herein which are obtainable or obtained by a method as disclosed herein.

Further, there is provided a method for providing an oral pouched nicotine product, said method comprising the steps of:

a) providing a filling material as described herein, and
b) enclosing said filling material in a saliva-permeable pouch.

There is also provided a use of a filling material as described herein for manufacturing of an oral pouched nicotine product.

The filling material disclosed herein may be produced by a method comprising the steps of:

a) providing nicotine-free particles formed from a carrier material comprising or consisting of microcrystalline cellulose,
b) providing a mixture comprising a nicotine source, a solvent such as water, and optionally an additive,
c) subjecting the particles of step a) to the mixture of step b) whereby part or all of the mixture is included in the nicotine-free particles from step a) thereby providing tobacco-free nicotine-containing particles,
d) optionally adding a flavouring agent to the particles resulting from step c),
e) optionally adding nicotine-free additional particles to the particles obtained in steps c) or d), and
f) optionally changing the moisture content of the particles in any one of steps a) to e).

The particles in steps a) to f) in the method described herein may independently be characterized by one or more of the following:

- a flow rate equal to or above about 2 grams/second and/or an angle of repose equal to or less than about 41°.

In particular, the particles in steps a) to f) in the method described herein may independently be characterized by one or more of the following:

a sphericity from about 0.8 to about 1.0,
a diameter from about 0.7 mm to about1.0 mm,
a d50 value from about 0.9 to about 1.0, and

a moisture content from about 8 wt% to about 15 wt% based one the total weight of the particles.

Further, the method described herein may lack particles that are smaller than about 0.7 mm, such as particles having a largest dimension that is below about 0.7 mm, such as particles having a diameter that is below about 0.7 mm. As a result, there is no need for removal of such small particles. This is a significant benefit since it simplifies the method and reduces problems associated with small particles such as dust. Accordingly, the method described herein may lack a particle removal step. Further, the method described herein may lack a drying step such as a drying involving or lacking heat. In this way, degradation of e.g. heat sensitive components and/or removal of volatile component is avoided. It will be appreciated that removal of a minor amount of solvent and/or volatile components, such as from about 1 wt% to about 25 wt% of the solvent and/or volatile components, is not regarded as drying. Advantageously, the moisture content of the particles is maintained or substantially maintained after manufacturing. This may be achieved by keeping the particles in a container, such as a pocket-sized container for snuff, at room temperature or refrigerated.

The tobacco-free particles and optionally the nicotine-free additional particles produced in accordance with the method described herein may be enclosed in a saliva-permeable pouch thereby providing an oral pouched nicotine product. Thus, there is provided a method for preparing an oral pouched nicotine product, the method comprising the steps of:

> a) providing tobacco-free nicotine-containing particles and optionally nicotine-free additional particles as described herein, and
>
> b) enclosing the particles from step a) in a saliva-permeable pouch.

There is also provided an oral pouched nicotine product as disclosed herein which is obtainable or obtained by a method as disclosed herein.

The oral pouched nicotine product as disclosed herein may have an oblong shape, such as a substantially rectangular shape (as seen from above when the product is placed on a planar surface). In such case, the longitudinal direction of the product corresponds to the length of the substantially rectangular product and the transverse direction of the product corresponds to the width of the substantially rectangular product. The pouch may comprise one or more seals such as a transverse seal and/or a longitudinal seal.

The total weight of the oral pouched nicotine product (including filling material and packaging material) may be within the range of from about 0.2 to about 2.5 g.

The oral pouched (i.e. portion-packed) nicotine products may be positioned randomly in a container or in a pattern, for instance as disclosed in WO 2012/069505. Alternatively or additionally, each oral pouched nicotine product may be placed in a sachet.

The oral pouched nicotine product as disclosed herein is intended for use in the oral cavity, such as by buccal placement (e.g. by placing the pouched product between the upper or lower gum and the lip or cheek), and may therefore be referred to as portion-packed (pouched) nicotine product for oral use, i.e. an oral pouched nicotine product. The oral pouched nicotine product is sized and configured to fit comfortably and discreetly in a user's mouth between the upper or lower gum and the lip or cheek.

The invention will be further described in the following examples, which do not limit the scope of the invention described in the claims.

EXAMPLES

**General**

Cellets 700 MCC spheres were supplied by Pharmatrans-Sanaq, Switzerland. These spheres are produced solely with microcrystalline cellulose. The powder in this Example section was obtained from Swedish Match and comprised microcrystalline cellulose, maltitol, hydroxypropyl cellulose powder, flavour additives, pH regulators and nicotine bitartrate. The microcrystalline cellulose and maltitol constitutet about 84 wt% based on the total weight of the powder. The nicotine bitartrate constituted about 2.5 wt% of the total weight of the powder. The powder had the following D values with respect to volume: d10= 125 micrometers, d50= 250 micrometers, d90= 400 micrometers.

**Abbreviations**

The following abbreviations are used throughout this document and their meaning is explained below.

mm    millimeter(s)
g      gram(s)

sec.    second(s)
h       hour(s)
min.    minute(s)
mcc     microcrystalline cellulose
mL      milliliter(s)

Example 1

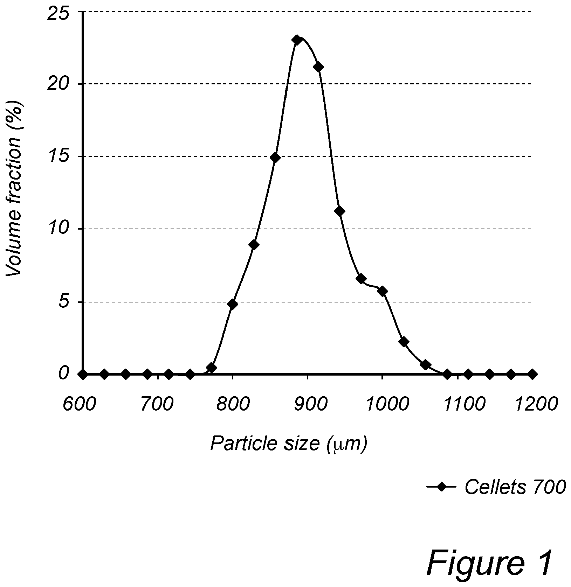

The sphericity and d50 value with respect to volume (see below) for the Cellets 700 were calculated with the aid of a QicPic instrument from 2012, Sympatec GmbH, ID No. 290-D, with Rodos/L dispersion line ID NO 214D and Vibri/L sample feeding ID NO 273. Three samples of 6 g each were analysed and an average calculated. The sphericity was found to be 0.93 with a standard deviation of 0.01. The d50 was found to be 890 $\mu$m. The particle size distribution is shown in Figure 1.

Example 2: Addition of nicotine to microcrystalline cellulose spheres

A solution was prepared from 17.5 grams of Nicotine bitartrate, 11.25 g of potassium carbonate and 66.25 g of water. The solution was mixed with 405 g grams of Cellets 700 MCC spheres (supplied by Pharmatrans-Sanaq, Switzerland) and allowed to stand for 24 hours at room temperature without stirring resulting in nicotine containing particles.

Example 3: Flow test

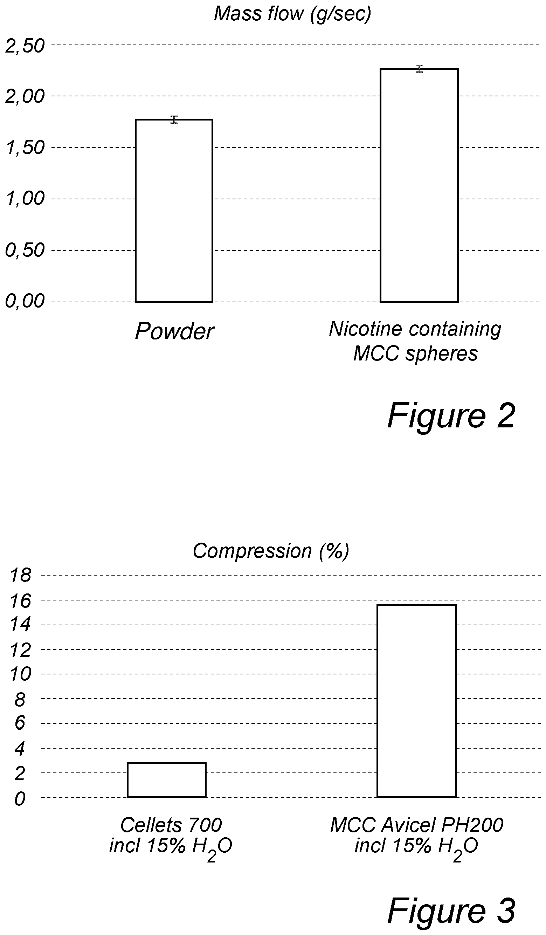

15 g of the powder from Swedish Match was poured through a glass funnel with 60 degree inclination and inner diameter of the funnel pipe of 5 mm. The time needed for the powder to pass the funnel was measured and this procedure was repeated five times. The flow rate was found to be 1.77 grams /second. The same procedure was done for the nicotine containing particles prepared in Example 2, resulting in a flow rate of 2.26 grams/second. Thus, the flow rate was higher for nicotine containing MCC spheres. The results are shown in Figure 2.

Example 4: Compression test

Samples of MCC spheres (Cellets 700) and of MCC powder (Avicel PH 200) were prepared using the following procedure: 212.5 g of MCC spheres and of MCC powder, respectively, was mixed with 37.5 g of water and stirred for 2 minutes. The samples were thereafter transferred to a sealed plastic bag and therein kept for 5 hours to allow the water to spread evenly. A 250 ml (volume before compression) sample from each bag was then transferred to a 250 ml measuring cylinder. The samples were compressed in a tapping apparatus (PharmaTest Touch) using 500 taps. Thereafter the compressed volume (volume after compression) was controlled. The compression of each sample was calculated according to the formula: Compression (%) = (Volume before compression (ml) - Volume after compression (ml) ) / Volume before compression (ml) * 100. The results showed that the compression when tapped was much greater for MCC powder than for MCC spheres. Thus, the sample comprising the MCC spheres resisted change of size better than the sample comprising the MCC powder.

Example 5: Storage stability of tobacco-free nicotine-containing particles

A solution was prepared from 18.0 grams of nicotine (supplied by Siegfried AG, Switzerland), 7.8 g of potassium carbonate (supplied by Alfa Aesar), 9.0 g tartaric acid, 54.0 g glycerol, 10 g sodium chloride, 0.51 g acesulfame K and 59.0 g of water. The solution was mixed with 841.7 g grams of Cellets 700 MCC spheres (supplied by Pharmatrans-Sanaq, Switzerland) and allowed to stand for 24 hours at room temperature without stirring whereby the solution was included in the Cellets 700 MCC spheres, forming tobacco-free nicotine-contained particles. The freshly prepared tobacco-free nicotine-containing particles appeared dry and were not sticky.

Visual inspection with the naked eye showed that the freshly prepared resulting tobacco-free nicotine-containing particles were white or almost white in appearance. The freshly prepared tobacco-free nicotine-containing particles were transferred to a sealed plastic bag and stored at room temperature for 17 weeks. Visual inspection with the naked eye after storage showed that the stored tobacco-free nicotine-containing particles had a beige to light brown color.

Since it is known that degraded nicotine is discolored it was concluded that part of the nicotine base was located on the outer surface of the tobacco-free nicotine-containing particles where it had been degraded during the storage.

**Claims**

1. An oral pouched nicotine product comprising a filling material and a saliva-permeable pouch of a packaging material enclosing the filling material,

   said filling material comprising:

   (a) tobacco-free nicotine-containing particles formed from a carrier material,

   said carrier material comprising microcrystalline cellulose,
   said tobacco-free nicotine-containing particles comprising a nicotine source,
   said tobacco-free nicotine-containing particles having
   a sphericity from 0.8 to 1.0,
   a diameter from 0.7 mm to 1.0 mm,
   a d50 value from 0.9 mm to 1.0 mm, and
   a moisture content from 8 wt% to 15 wt% based one the total weight of the tobacco-free nicotine-containing particles, and
   (b) optionally a tobacco material present in an amount within the range of from 0.05 wt% to 10 wt%, based on the total weight of said filling material
   with the proviso that said oral pouched nicotine product is free from or substantially free from a constituent comprising a coating comprising a nicotine source and a binder.

2. The oral pouched nicotine product according to claim 1, wherein the carrier material consists of microcrystalline cellulose.

3. The oral pouched nicotine product according to claim 1 or 2, which is free from tobacco material or which comprises tobacco material within the range of from 0.05 wt% to 10 wt%, such as from 1 wt% to 5 wt%, such as from 0.2 wt% to 1 wt%, based on the total weight of said filling material.

4. The oral pouched nicotine product according to any one of the preceding claims, wherein the nicotine source is absorbed in the carrier material.

5. The oral pouched nicotine product according to any one of the preceding claims, wherein at least part of the nicotine source is present on an outer surface of the tobacco-free nicotine-containing particles.

6. The oral pouched nicotine product according to any one of the preceding claims, wherein the tobacco-free nicotine-containing particles are non-dried.

7. The oral pouched nicotine product according to any one of the preceding claims, wherein the filling material further comprises a pH adjusting agent such as $Na_2CO_3$, $K_2CO_3$, NaHCOs and/or $KHCO_3$, such as wherein the pH adjusting agent is included in the tobacco-free nicotine-containing particles.

8. The oral pouched nicotine product according to any one of claims 1-7, wherein the tobacco-free nicotine containing particles are **characterized by** one or more of the following:

   - a flow rate equal to or above 2 grams/second and/or an angle of repose equal to or less than 41°.

9. The oral pouched nicotine product according to any one of the preceding claims, wherein the filling material further comprises nicotine-free additional particles comprising:

   (i) a carrier material comprising microcrystalline cellulose,
   (ii) optionally a pH adjusting agent included in said carrier material,

   said nicotine free additional particles having a sphericity from 0.8 to 1.0, and a diameter from 0.1 mm to 2.0 mm.

10. A method for preparing an oral pouched nicotine product as defined in any one of the preceding claims, said method comprising the steps of:

a) providing a filling material as defined in any one of claims 1-9, and
b) enclosing said filling material in a saliva-permeable pouch.

**11.** Use of a filling material as defined in any one of claims 1-9 for manufacturing of an oral pouched nicotine product.

**12.** A method for preparing a filling material as defined in any one of claims 1-9, the method comprising the steps of:

a) providing nicotine-free particles formed from a carrier material comprising or consisting of microcrystalline cellulose,
b) providing a mixture comprising a nicotine source, a solvent such as water, and optionally an additive,
c) subjecting the particles of step a) to the mixture of step b) whereby part or all of the mixture is included in the nicotine-free particles from step a) thereby providing tobacco-free nicotine-containing particles,
d) optionally adding a flavouring agent to the particles resulting from step c),
e) optionally adding nicotine-free additional particles to the particles obtained in steps c) or d), and
f) optionally changing the moisture content of the particles in any one of steps a) to e).

**13.** The method according to claim 12, which lacks particles that are smaller than 0.7 mm.

**14.** The method according to claim 12 or 13, which lacks a particle removal step and/or lacks a drying step.

**15.** A method for preparing an oral pouched nicotine product, the method comprising the steps of:

a) providing tobacco-free nicotine-containing particles and optionally nicotine-free additional particles produced in accordance with any one of claims 12-14, and
b) enclosing the particles from step a) in a saliva-permeable pouch.


**Patentansprüche**

**1.** Orales Nikotinprodukt im Beutel, umfassend ein Füllmaterial und einen speicheldurchlässigen Beutel eines Verpackungsmaterials, das das Füllmaterial umschließt, das Füllmaterial umfassend:

(a) tabakfreie nikotinhaltige Partikel, die aus einem Trägermaterial ausgebildet sind,

das Trägermaterial umfassend mikrokristalline Cellulose,
die tabakfreien nikotinhaltigen Partikel umfassend eine Nikotinquelle,
wobei die tabakfreien nikotinhaltigen Partikel aufweisen:

eine Sphärizität von 0,8 bis 1,0,
einen Durchmesser von 0,7 mm bis 1,0 mm,
einen d50-Wert von 0,9 mm bis 1,0 mm, und
einen Feuchtigkeitsgehalt von 8 Gew.-% bis 15 Gew.-%, bezogen auf das Gesamtgewicht der tabakfreien nikotinhaltigen Partikel, und

(b) optional ein Tabakmaterial, das in einer Menge innerhalb des Bereichs von 0,05 Gew.-% bis 10 Gew.-% vorhanden ist, bezogen auf das Gesamtgewicht des Füllmaterials
mit der Maßgabe, dass das orale Nikotinprodukt im Beutel frei von oder im Wesentlichen frei von einem Bestandteil ist, umfassend eine Beschichtung, umfassend eine Nikotinquelle und ein Bindemittel.

**2.** Orales Nikotinprodukt im Beutel nach Anspruch 1, wobei das Trägermaterial aus mikrokristalliner Cellulose besteht.

**3.** Orales Nikotinprodukt im Beutel nach Anspruch 1 oder 2, das frei von Tabakmaterial ist oder das Tabakmaterial innerhalb des Bereichs von 0,05 Gew.-% bis 10 Gew.-%, wie von 1 Gew.-% bis 5 Gew.-%, wie von 0,2 Gew.-% bis 1 Gew.-%, bezogen auf das Gesamtgewicht des Füllmaterials, umfasst.

**4.** Orales Nikotinprodukt im Beutel nach einem der vorstehenden Ansprüche, wobei die Nikotinquelle in dem Trägermaterial absorbiert wird.

5. Orales Nikotinprodukt im Beutel nach einem der vorstehenden Ansprüche, wobei mindestens ein Teil der Nikotinquelle auf einer Außenoberfläche der tabakfreien nikotinhaltigen Partikel vorhanden ist.

6. Orales Nikotinprodukt im Beutel nach einem der vorstehenden Ansprüche, wobei die tabakfreien nikotinhaltigen Partikel nicht getrocknet sind.

7. Orales Nikotinprodukt im Beutel nach einem der vorstehenden Ansprüche, wobei das Füllmaterial ferner ein pH-Wert-Einstellmittel wie $Na_2CO_3$, $K_2CO_3$, NaHCOs und/oder KHCOs umfasst, wobei das pH-Wert-Einstellmittel in den tabakfreien nikotinhaltigen Partikeln eingeschlossen ist.

8. Orales Nikotinprodukt im Beutel nach einem der Ansprüche 1 bis 7, wobei die tabakfreien nikotinhaltigen Partikel **gekennzeichnet sind durch** eines oder mehrere der Folgenden:

   - eine Durchflussrate, die gleich oder über 2 Gramm/Sekunde ist und/oder einen Schüttwinkel, der gleich oder kleiner als 41 ° ist.

9. Orales Nikotinprodukt im Beutel nach einem der vorstehenden Ansprüche, wobei das Füllmaterial ferner nikotinfreie zusätzliche Partikel umfasst, umfassend:

   (i) ein Trägermaterial, umfassend mikrokristalline Cellulose,
   (ii) optional ein pH-Wert-Einstellmittel, das in dem Trägermaterial eingeschlossen ist,

   wobei die nikotinfreien zusätzlichen Partikel eine Sphärizität von 0,8 bis 1,0 und einen Durchmesser von 0,1 mm bis 2,0 mm aufweisen.

10. Verfahren zum Herstellen eines oralen Nikotinprodukts im Beutel nach einem der vorstehenden Ansprüche, das Verfahren umfassend die Schritte:

    a) Bereitstellen eines Füllmaterials nach einem der Ansprüche 1 bis 9 und
    b) Umschließen des Füllmaterials in einem speicheldurchlässigen Beutel.

11. Verwenden eines Füllmaterials nach einem der Ansprüche 1 bis 9 zum Fertigen eines oralen Nikotinprodukts im Beutel.

12. Verfahren zum Herstellen eines Füllmaterials nach einem der Ansprüche 1 bis 9, das Verfahren umfassend die Schritte:

    a) Bereitstellen von nikotinfreien Partikeln, die aus einem Trägermaterial ausgebildet sind, umfassend oder bestehend aus mikrokristalliner Cellulose,
    b) Bereitstellen einer Mischung, umfassend eine Nikotinquelle, ein Lösungsmittel wie Wasser und optional ein Additiv,
    c) Unterziehen der Partikel von Schritt a) der Mischung von Schritt b), wobei ein Teil der oder die gesamte Mischung in den nikotinfreien Partikeln von Schritt a) eingeschlossen ist, wodurch tabakfreie nikotinhaltige Partikel bereitgestellt werden,
    d) optional Hinzufügen eines Aromatisierungsmittels zu den Partikeln, die aus Schritt c) resultieren,
    e) optional Hinzufügen von nikotinfreien zusätzlichen Partikeln zu den in den Schritten c) oder d) erhaltenen Partikeln, und
    f) optional Ändern des Feuchtigkeitsgehalts der Partikel in einem beliebigen der Schritte a) bis e).

13. Verfahren nach Anspruch 12, bei dem es an Partikel fehlt, die kleiner als 0,7 mm sind.

14. Verfahren nach Anspruch 12 oder 13, bei dem es an einem Partikelentfernungsschritt fehlt und/oder einem Trocknungsschritt fehlt.

15. Verfahren zum Herstellen eines oralen Nikotinprodukts im Beutel, das Verfahren umfassend die Schritte:

    a) Bereitstellen von tabakfreien nikotinhaltigen Partikeln und optional nikotinfreien zusätzlichen Partikeln, die nach einem der Ansprüche 12 bis 14 produziert werden, und

b) Umschließen der Partikel von Schritt a) in einem speicheldurchlässigen Beutel.

**Revendications**

1. Produit de nicotine oral en sachet comprenant un matériau de remplissage et un sachet perméable à la salive d'un matériau de conditionnement enfermant le matériau de remplissage, ledit matériau de remplissage comprenant :

    (a) des particules contenant de la nicotine dépourvues de tabac formées à partir d'un matériau véhicule,

    ledit matériau véhicule comprenant de la cellulose microcristalline,
    lesdites particules contenant de la nicotine dépourvues de tabac comprenant une source de nicotine,
    lesdites particules contenant de la nicotine dépourvues de tabac ayant
    une sphéricité allant de 0,8 à 1,0,
    un diamètre allant de 0,7 mm à 1,0 mm,
    une valeur d50 allant de 0,9 mm à 1,0 mm, et
    une teneur en humidité allant de 8 % en poids à 15 % en poids en fonction du poids total des particules contenant de la nicotine dépourvues de tabac, et

    (b) facultativement un matériau de tabac présent en une quantité dans la plage allant de 0,05 % en poids à 10 % en poids, en fonction du poids total dudit matériau de remplissage
    à condition que ledit produit de nicotine oral en sachet soit dépourvu ou sensiblement dépourvu d'un constituant comprenant un revêtement comprenant une source de nicotine et un liant.

2. Produit de nicotine oral en sachet selon la revendication 1, dans lequel le matériau véhicule est constitué de cellulose microcristalline.

3. Produit de nicotine oral en sachet selon la revendication 1 ou 2, qui est dépourvu de matériau de tabac ou qui comprend un matériau de tabac dans la plage allant de 0,05 % en poids à 10 % en poids, telle que de 1 % en poids à 5 % en poids, telle que de 0,2 % en poids à 1 % en poids, en fonction du poids total dudit matériau de remplissage.

4. Produit de nicotine oral en sachet selon l'une quelconque des revendications précédentes, dans lequel la source de nicotine est absorbée dans le matériau véhicule.

5. Produit de nicotine oral en sachet selon l'une quelconque des revendications précédentes, dans lequel au moins une partie de la source de nicotine est présente sur une surface externe des particules contenant de la nicotine dépourvues de tabac.

6. Produit de nicotine oral en sachet selon l'une quelconque des revendications précédentes, dans lequel les particules contenant de la nicotine dépourvues de tabac sont non séchées.

7. Produit de nicotine oral en sachet selon l'une quelconque des revendications précédentes, dans lequel le matériau de remplissage comprend en outre un agent d'ajustement de pH tel que $Na_2CO_3$, $K_2CO_3$, NaHCOs et/ou KHCOs, tel que dans lequel l'agent d'ajustement de pH est inclus dans les particules contenant de la nicotine dépourvues de tabac.

8. Produit de nicotine oral en sachet selon l'une quelconque des revendications 1 à 7, dans lequel les particules contenant de la nicotine dépourvues de tabac sont **caractérisées par** un ou plusieurs de ce qui suit :

    - un débit égal ou supérieur à 2 grammes/seconde et/ou un angle de repos égal ou inférieur à 41°.

9. Produit de nicotine oral en sachet selon l'une quelconque des revendications précédentes, dans lequel le matériau de remplissage comprend en outre des particules supplémentaires dépourvues de nicotine comprenant :

    (i) un matériau véhicule comprenant de la cellulose microcristalline,
    (ii) facultativement un agent d'ajustement de pH inclus dans ledit matériau véhicule,

    lesdites particules supplémentaires dépourvues de nicotine ont une sphéricité allant de 0,8 à 1,0, et un diamètre allant de 0,1 mm à 2,0 mm.

10. Procédé de préparation d'un produit de nicotine oral en sachet tel que défini dans l'une quelconque des revendications précédentes, ledit procédé comprenant les étapes consistant à :

   a) fournir un matériau de remplissage tel que défini dans l'une quelconque des revendications 1 à 9, et
   b) enfermer ledit matériau de remplissage dans un sachet perméable à la salive.

11. Utilisation d'un matériau de remplissage tel que défini dans l'une quelconque des revendications 1 à 9 pour la fabrication d'un produit de nicotine oral en sachet.

12. Procédé de préparation d'un matériau de remplissage tel que défini dans l'une quelconque des revendications 1 à 9, le procédé comprenant les étapes consistant à :

   a) fournir des particules dépourvues de nicotine formées à partir d'un matériau véhicule comprenant ou constitué par de la cellulose microcristalline,
   b) fournir un mélange comprenant une source de nicotine, un solvant tel que de l'eau, et facultativement un additif,
   c) soumettre les particules de l'étape a) au mélange de l'étape b) moyennant quoi une partie ou la totalité du mélange est incluse dans les particules dépourvues de nicotine provenant de l'étape a) ce qui fournit des particules contenant de la nicotine dépourvues de tabac,
   d) facultativement ajouter un agent aromatisant aux particules résultant de l'étape c),
   e) facultativement ajouter des particules supplémentaires dépourvues de nicotine aux particules obtenues aux étapes c) ou d), et
   f) facultativement changer la teneur en humidité des particules dans l'une quelconque des étapes a) à e).

13. Procédé selon la revendication 12, qui est exempt de particules qui sont plus petites que 0,7 mm.

14. Procédé selon la revendication 12 ou 13, qui est exempt d'une étape d'élimination de particules et/ou est exempt d'une étape de séchage.

15. Procédé de préparation d'un produit de nicotine oral en sachet, le procédé comprenant les étapes consistant à :

   a) fournir des particules contenant de la nicotine dépourvues de tabac et facultativement des particules supplémentaires dépourvues de nicotine selon l'une quelconque des revendications 12 à 14, et
   b) enfermer les particules provenant de l'étape a) dans un sachet perméable à la salive.

Figure 1

Mass flow (g/sec)

Figure 2

Compression (%)

Figure 3

**Figure 4a**

**Figure 4b**

$$S = \frac{P_{EQPC}}{P_{real}} \qquad \frac{2\sqrt{\pi \cdot A}}{P_{real}}$$

**Figure 5**

*Figure 6*

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2177213 A
- EP 1338288 A
- US 20190083393 A
- WO 2017125405 A
- WO 2012069505 A

**Non-patent literature cited in the description**

- Total moisture determination. *Federal Register,* 07 January 2009, vol. 74 (4), 712-719
- Moisture in Tobacco. Official Methods of Analysis. AOAC (Association of Official Analytical Chemics), 1990, vol. 966